# FASCICULE DE BREVET EUROPEEN

(11) **EP 2 016 838 B1**
(45) Date de publication et mention de la délivrance du brevet: **31.05.2017**
(21) Numéro de dépôt: 07360026.4
(22) Date de dépôt: 18.06.2007
(51) Int. Cl.: A23L 33/185, A23L 33/19, A23L 33/17

(54) **Supplément alimentaire**
Nahrungsergänzung
Food supplement

(43) Date de publication de la demande: 21.01.2009
(73) Titulaire: ET & DS Company Ltd, 3105 Limassol (CY)
(72) Inventeur: Chossade, Christian, 86120 MORTON (FR)
(74) Mandataire: Littolff, Denis

(56) Documents cités:
- WO-A-2006/060414
- WO-A-2007/053619
- US-A- 4 079 125
- US-A- 5 569 458
- US-A- 5 902 617
- US-A1- 2004 253 295

## Description

La présente invention concerne la digestion, à titre essentiel des protéines de lait, mais également d'autres protéines absorbées en quantité importante et de façon répétée. Elle a trait à un supplément alimentaire favorisant une bonne ingestion et une bonne digestion de protéines animales et/ou végétales, et constitué d'un mélange d'enzymes comprenant l'amylase, la lactase et la bromélaïne.

L'emploi d'enzymes digestives dans des compositions thérapeutiques ou compléments alimentaires est connu. Par exemple le document WO2007/053619 décrit une composition, pour le traitement de l'insuffisance pancréatique d'enzymes, comprenant une lipase, une amylase ou une amyloglucosidase et au moins une protéase. Le document US4079125 décrit une composition, pour traiter les désordres digestifs, comprenant des enzymes pancréatiques, et le document US5569458 décrit une composition, pour améliorer l'absorption de nutriments, comprenant des vitamines, des minéraux et des enzymes pancréatiques.

La digestion de la protéine de lait pose en particulier, à de nombreux égards, problème. Ainsi, certaines personnes ne sont pas en mesure de conserver au sein de leur organisme une enzyme particulière, la lactase qui sert à digérer le lactose contenu dans les produits laitiers. Ils sont de fait intolérants au lactose. Lorsque ces personnes consomment des produits laitiers, elles souffrent de maux tels que vomissements, ballonnements, halitoses, crampes ou d'autres troubles intestinaux du type diarrhées, etc...

En fait, la lactase est déficiente chez une grande partie de la population adulte mondiale. Cette enzyme, présente chez les nouveaux-nés pour la digestion du lait maternel, disparaît peu à peu après le sevrage. Continuer à consommer du lait revient à maintenir la lactase faiblement active, mais c'est aller à l'encontre de la nature. On force la production d'une enzyme en principe prévue pour disparaître. de la même manière que d'autres protéines absorbées soit en quantités importantes soit sans lipides et/ou glucides. Cela provoque les symptômes décrits ci-dessus. La production insuffisante de lactase par l'organisme outre les conséquences décrites ci-dessus, peut également engendrer des troubles plus graves.

Il ne suffit donc pas de supplémenter l'organisme en lactase en vue d'éviter les troubles digestifs décrits ci-dessus, car un apport mal dosé sur une période trop longue génère d'autres risques, par exemple de mauvaise circulation sanguine.

En référence au problème lié à la digestion que résout l'invention, l'amylase - second constituant de l'invention - est une enzyme permettant notamment la digestion de l'amidon et des dextrines en les transformant en sucres réducteurs assimilables. Elle est sécrétée par le pancréas et les glandes salivaires. Il est possible d'augmenter la quantité d'amylase dans l'organisme lorsque l'une ou l'autre de ces glandes est défectueuse.

L'un de ses intérêts majeurs dans la perspective de favoriser la digestion est qu'elle augmente la quantité de salive dans la bouche et permet le démarrage d'un travail de réduction des aliments. Elle évite la sensation de "poids mort" due aux protéines ingérées.

Une absorption trop importante d'amylase aboutit cependant à une augmentation anormale du taux des triglycérides. Cela peut provoquer des pancréatites chroniques, des cholécystites, des kystes, des perforations d'ulcère du duodénum ou des péritonites, notamment en cas d'absorption d'enzymes animales. Son dosage est par conséquent important.

Toujours dans la même perspective digestive, le troisième constituant du supplément alimentaire de l'invention, la bromélaïne par ailleurs connue et utilisée dans le traitement des maladies veineuses, des contusions, de l'arthrite, de la polyarthrite rhumatoïde et de la goutte, permet aussi la réduction totale des protéines et de ses composants, en agissant cependant moins vite que la lactose et l'amylase. Elle complète par conséquent leur action. On l'utilise également en Europe pour favoriser le rétablissement après une opération ou des blessures sportives ou pour traiter des sinusites ou des phlébites.

A forte dose, elle provoque toutefois des effets secondaires tels que vomissements et insuffisance rénale sévère, et il convient dès lors de mesurer soigneusement son dosage.

Le but de la présente invention vise à réaliser un supplément alimentaire assurant d'une part une absorption buccale correcte et d'autre part une digestion sans troubles, en évitant cependant les effets secondaires potentiels dus à ses constituants.

En d'autres termes, le supplément alimentaire de l'invention doit favoriser une bonne ingestion et une bonne digestion de protéines animales et/ou végétales. A cet effet, selon l'invention, le mélange d'enzymes constitué d'amylase, de lactase et de bromélaïne, doit respecter le dosage pondéral suivant :
- 15 mg à 90 mg pour l'amylase ;
- 18 mg à 90 mg pour la lactase ; et
- 6 mg à 80 mg pour la bromélaïne.

L'utilisation de ces enzymes est principalement intéressante dans la mesure où elle permet d'accélérer les réactions chimiques et biochimiques qui se produisent lors de la digestion. Ces enzymes ont ainsi un rôle de catalyseur dans l'organisme humain.

Le supplément alimentaire de l'invention est indiqué en cas de difficultés liées à la digestion de tout type de protéines, indépendamment de l'origine végétale ou animale de celles-ci. Il permet d'abord d'augmenter la teneur en lactase de l'organisme aux fins d'une bonne digestion, mais aussi de permettre une mise en bouche différente (ingestion buccale des aliments) en associant la lactase à l'amylase.

Enfin, l'association de la bromélaïne à l'amylase et à la lactase favorise la digestion sans effets secondaires gênants ou non souhaités. Le dosage de la lactase est particulièrement important car un tel dosage que l'on peut qualifier de faible permet d'éviter des effets néfastes sur la circulation sanguine, même en cas d'ingestion sur une longue période. Les effets secondaires le cas échéant provoqués par un fort dosage en lactase sont ainsi évités.

Le dosage précité de l'amylase, entre 15 et 90 mg, permet également d'éviter tout risque de complication lié à une prise prolongée, tout en apportant la contribution expliquée auparavant à la digestion des protéines.

L'utilisation de la bromélaïne, à raison d'une quantité comprise entre 6 mg et 80 mg, permet d'apporter un bénéfice significatif à la digestion et n'engendre aucun effet secondaire en cas d'ingestion sur une période longue.

De nombreuses applications du supplément alimentaire de l'invention sont possibles.

Ainsi, selon un exemple, pour un supplément alimentaire notamment destiné à des sportifs, les quantités des différentes enzymes peuvent plus précisément être :
- 30 mg pour l'amylase ;
- 40 mg pour la lactase ; et
- 15 mg pour la bromélaïne.

Pour une autre application destinée à une utilisation plus diététique, les quantités prévues sont de l'ordre de :
- 40 mg pour l'amylase ;
- 60 mg pour la lactase ; et
- 25 mg pour la bromélaïne.

Selon l'invention, le supplément alimentaire peut se présenter sous toutes les formes galéniques possibles, à savoir : poudre ou granulé à réhydrater ou à consommer comme tel, comprimé, tablette, gélule, mais également sous présentation liquide ou pâteuse. Ces formes ou présentations ne sont pas exhaustives.

L'invention concerne par ailleurs un produit alimentaire concentré protéiné et supplémenté au moyen d'un supplément alimentaire tel que présenté ci-dessus. De préférence, ledit concentré protéiné présente une teneur en protéines supérieure à 82 %.

Un tel produit alimentaire concentré et supplémenté permet de faciliter la digestion des aliments hyperprotéinés de tout type. Il favorise secondairement le drainage et l'élimination des graisses chez les sportifs, ainsi que l'enrichissement de leur masse musculaire.

Il permet par ailleurs d'obtenir des résultats spectaculaires pour résorber les graisses chez les personnes obèses.

Dans un tel produit, les protéines concentrées peuvent être d'origine animale. Selon une variante, elles peuvent également être d'origine végétale, ou encore résulter d'un mélange des deux.

De préférence, selon l'invention, on utilise de l'amylase d'origine végétale et de la lactase d'origine végétale et/ou animale.

Selon les essais réalisés, l'association de l'amylase, de la lactase et de la bromélaïne avec des dosages compris dans les fourchettes indiquées précédemment à savoir 15 à 90 mg pour l'amylase, 18 à 90 mg pour la lactase, et 6 à 80 mg pour la bromélaïne permet d'obtenir un résultat significatif et fiable dans l'amélioration de la digestion des protéines. L'association de ces dosages -que l'on peut qualifier de faibles- d'enzymes est donc, contre toute attente, particulièrement indiquée dans le traitement des difficultés digestives.

Les résultats lié à l'association de ces trois enzymes à faible dosage sont d'autant plus remarquables que leur utilisation n'était jusqu'ici connue qu'à des dosages forts ou élevés pour l'obtention d'un résultat significatif dans la digestion, au risque d'effets secondaires gênants, voire dangereux dans certains cas. Les résultats particulièrement intéressants obtenus dans la digestion des protéines ne peuvent en tout état de cause pas être observés avec une prise de l'une quelconque des enzymes précédentes de façon individuelle avec un dosage tel qu'indiqué.

Un avantage indéniable du supplément alimentaire conforme à l'invention réside donc en grande partie dans l'interaction de l'amylase, de la lactase et de la bromélaïne, chacune faiblement dosée.

## Revendications

1. Supplément alimentaire favorisant une bonne ingestion et une bonne digestion de protéines animales et/ou végétales, constitué d'un mélange d'enzymes constitué d'amylase, de lactase et de bromélaïne,
leur dosage pondéral étant le suivant :
- 15 mg à 90 mg pour l'amylase ;
- 18 mg à 90 mg pour la lactase ; et
- 6 mg à 80 mg pour la bromélaïne.

2. Supplément alimentaire selon la revendication 1, destiné à des sportifs, les quantités des différentes enzymes étant :
- 30 mg pour l'amylase ;
- 40 mg pour la lactase ; et
- 15 mg pour la bromélaïne.

3. Supplément alimentaire selon la revendication 1, destiné à une utilisation diététique, les quantités étant :
- 40 mg pour l'amylase ;
- 60 mg pour la lactase ; et
- 25 mg pour la bromélaïne.

4. Supplément alimentaire selon l'une quelconque des revendications 1 à 3, présenté sous une des formes galéniques suivantes :
- gélules;
- poudre ou granulés à réhydrater ;
- comprimés ou tablettes ;
- présentation liquide ou pâteuse.

5. Produit alimentaire concentré protéiné, supplémenté au moyen d'un supplément alimentaire conforme à l'une quelconque des revendications 1 à 4, **caractérisé en ce qu'**il présente une teneur en protéines supérieure à 82 %.

6. Produit alimentaire concentré protéiné et supplémenté selon la revendication 5, dans lequel les protéines sont d'origine animale.

7. Produit alimentaire concentré, protéiné et supplémenté selon la revendication 5, dans lequel les protéines sont d'origine végétale.

## Patentansprüche

1. Nahrungsergänzung, die eine gute Aufnahme und eine gute Verdauung von tierischen und/oder pflanzlichen Proteinen fördert, gebildet von einem Enzymgemisch, gebildet von Amylase, Laktase und Bromelain,
wobei ihre Gewichtsdosierung folgende ist:
- 15 mg bis 90 mg Amylase,
- 18 mg bis 90 mg Laktase und
- 6 mg bis 80 mg Bromelain.

2. Nahrungsergänzung nach Anspruch 1 für Sportler, wobei die Mengen der verschiedenen Enzyme sind:
- 30 mg Amylase,
- 40 mg Laktase und
- 15 mg Bromelain.

3. Nahrungsergänzung nach Anspruch 1 für eine diätische Anwendung, wobei die Mengen sind:
- 40 mg Amylase,
- 60 mg Laktase und
- 25 mg Bromelain.

4. Nahrungsergänzung nach einem der Ansprüche 1 bis 3, präsentiert in einer der folgenden galenischen Formen:
- Kapseln,
- Pulver oder Granulat zum Rehydrieren,
- Tabletten oder Pillen,
- flüssige oder pastöse Präsentation.

5. Konzentriertes proteinreiches und mit einer Nahrungsergänzung nach einem der Ansprüche 1 bis 4 supplementiertes Lebensmittelprodukt, **dadurch gekennzeichnet dass** es einen Proteingehalt von über 82 % aufweist.

6. Konzentriertes proteinreiches und supplementiertes Lebensmittelprodukt nach Anspruch 5, wobei die Proteine tierischen Ursprungs sind.

7. Konzentriertes proteinreiches und supplementiertes Lebensmittelprodukt nach Anspruch 5, wobei die Proteine pflanzlichen Ursprungs sind.

## Claims

1. A food supplement promoting good ingestion and good digestion of animal and/or plant proteins, consisting of a mixture of enzymes consisting of amylase, a lactase and of bromelain,
their weight dosage being the following:
- 15 mg to 90 mg for the amylase;
- 18 mg to 90 mg for the lactase; and
- 6 mg to 80 mg for the bromelain.

2. The food supplement according to claim 1, intended for sportsmen, the amounts of the different enzymes being:
- 30 mg for the amylase;
- 40 mg for the lactase; and
- 15 mg for the bromelain.

3. The food supplement according to claim 1, intended for dietary use, the amounts being:
- 40 mg for the amylase;
- 60 mg for the lactase; and
- 25 mg for the bromelain.

4. The food supplement according to any of claims 1 to 3, presented in one of the following galenic forms:
- gelatin capsules;
- powder or granules to be rehydrated;
- pellets or tablets;
- a liquid or slurry presentation.

5. A protein-containing concentrated food product, are supplemented by means of a food supplement compliant with any of claims 1 to 4, **characterized in that** it has a protein content of more than 82%.

6. The protein-containing and supplemented concentrated food product according to claim 5, wherein the proteins are of animal origin.

7. The protein-containing and supplemented concentrated food product according to claim 5, wherein the proteins are of plant origin.
